# EUROPEAN PATENT SPECIFICATION

(11) **EP 3 280 430 B1**
(45) Date of publication and mention of the grant of the patent: **08.04.2020**
(21) Application number: 16725038.0
(22) Date of filing: 08.04.2016
(51) Int. Cl.: A61K 38/04, A61P 25/00

(54) **QBP1-PEPTIDE FOR USE IN THE PREVENTION OR TREATMENT OF POST-TRAUMATIC STRESS DISORDER, ACUTE STRESS SYNDROME AND GENERAL ADAPTATION SYNDROME**
QBP1-PEPTID ZUR VERWENDUNG BEI DER VORBEUGUNG UND BEHANDLUNG POSTTRAUMATISCHER BELASTUNGSSTÖRUNG, AKUTEN BELASTUNGSSYNDROMS UND ALLGEMEINEN ANPASSUNGSSYNDROMS
PEPTIDE QBP1 POUR SON UTILISATION DANS LA PRÉVENTION ET LE TRAITEMENT DU TROUBLE DE STRESS POST-TRAUMATIQUE, SYNDROME DE STRESS AIGU ET SYNDROME D'ADAPTATION GÉNÉRALE

(30) Priority: 10.04.2015 EP 15382176
(43) Date of publication of application: 14.02.2018
(73) Proprietor: Consejo Superior De Investigaciones Científicas, 28006 Madrid (ES)
(72) Inventor: CARRIÓN VÁZQUEZ, Mariano Sixto, 28002 Madrid (ES); HERVÁS MILÁN, Rubén, 28002 Madrid (ES)
(74) Representative: Pons Ariño, Angel
(86) International application number: PCT/EP2016/057801
(87) International publication number: WO 2016/162506

(56) References cited:
- WO-A1-2014/136118
- RUBÉN HERVÁS ET AL: "Common Features at the Start of the Neurodegeneration Cascade", PLOS BIOLOGY, vol. 10, no. 5, 1 January 2012 (2012-01-01), page e1001335, XP055202153, ISSN: 1544-9173, DOI: 10.1371/journal.pbio.1001335
- Y. NAGAI: "Prevention of polyglutamine oligomerization and neurodegeneration by the peptide inhibitor QBP1 in Drosophila", HUMAN MOLECULAR GENETICS, vol. 12, no. 11, 1 June 2003 (2003-06-01), pages 1253-1259, XP055202141, ISSN: 0964-6906, DOI: 10.1093/hmg/ddg144
- H. AKIKO POPIEL ET AL: "The Aggregation Inhibitor Peptide QBP1 as a Therapeutic Molecule for the Polyglutamine Neurodegenerative Diseases", JOURNAL OF AMINO ACIDS, vol. 378, no. 6555, 1 January 2011 (2011-01-01), pages 403-10, XP055202129, DOI: 10.1038/nm0798-822
- TOMITA K ET AL: "Structure-activity relationship study on polyglutamine binding peptide QBP1", BIOORGANIC & MEDICINAL CHEMISTRY, PERGAMON, GB, vol. 17, no. 3, 1 February 2009 (2009-02-01), pages 1259-1263, XP025925753, ISSN: 0968-0896, DOI: 10.1016/J.BMC.2008.12.018 [retrieved on 2009-01-31]
- GRÄFF JOHANNES ET AL: "Epigenetic Priming of Memory Updating during Reconsolidation to Attenuate Remote Fear Memories", CELL, CELL PRESS, US, vol. 156, no. 1, 16 January 2014 (2014-01-16), pages 261-276, XP028811722, ISSN: 0092-8674, DOI: 10.1016/J.CELL.2013.12.020
- VIEWEG W V R ET AL: "Posttraumatic Stress Disorder: Clinical Features, Pathophysiology, and Treatment", AMERICAN JOURNAL OF MEDICINE, EXCERPTA MEDICA, INC, UNITED STATES, vol. 119, no. 5, 1 May 2006 (2006-05-01), pages 383-390, XP027910827, ISSN: 0002-9343 [retrieved on 2006-05-01]
- H. REN ET AL: "Amino Acid Sequence Requirements of Peptides That Inhibit Polyglutamine-Protein Aggregation and Cell Death", BIOCHEMICAL AND BIOPHYSICAL RESEARCH COMMUNICATIONS, vol. 288, no. 3, 1 November 2001 (2001-11-01), pages 703-710, XP055202316, ISSN: 0006-291X, DOI: 10.1006/bbrc.2001.5783

### SEQUENCE LISTING

<110> Consejo Superior de Investigaciones Cientificas
<120> Use of QBP1 peptide for the inhibition of memory consolidation
<130> PCT1641.1052
<150> EP15382176
   <151> 10-04-2015
<160> 7
<170> PatentIn version 3.5
<210> 1
   <211> 8
   <212> PRT
   <213> Artificial Sequence
<220>
   <223> Fragment of QBP1
<400> 1
   Trp Lys Trp Trp Pro Gly Ile Phe
   1               5
<210> 2
   <211> 11
   <212> PRT
   <213> Artificial Sequence
<220>
   <223> QBP1
<400> 2
   Ser Asn Trp Lys Trp Trp Pro Gly Ile Phe Asp
   1 5 10
<210> 3
   <211> 11
   <212> PRT
   <213> Artificial Sequence
<220>
   <223> Tat 47-57
<400> 3
   Tyr Gly Arg Lys Lys Arg Arg Gln Arg Arg Arg
   1 5 10
<210> 4
   <211> 13
   <212> PRT
   <213> Artificial Sequence
<220>
   <223> Tat 48-60
<400> 4
   Gly Arg Lys Lys Arg Arg Gln Arg Arg Arg Pro Pro Gln
   1 5 10
<210> 5
   <211> 16
   <212> PRT
   <213> Artificial Sequence
<220>
   <223> Antp
<400> 5
   Arg Gln Ile Lys Ile Trp Phe Gln Asn Arg Arg Met Lys Trp Lys Lys
   1 5 10 15
<210> 6
   <211> 21
   <212> PRT
   <213> Artificial Sequence
<220>
   <223> Pep-1
<400> 6
   Lys Glu Thr Trp Trp Glu Thr Trp Trp Thr Glu Trp Ser Gln Pro Lys
   1               5                   10                  15
   Lys Lys Arg Lys Val
               20
<210> 7
   <211> 8
   <212> PRT
   <213> Artificial Sequence
<220>
   <223> Scramble
<400> 7
   Trp Pro Ile Trp Lys Gly Trp Phe
   1               5

## Description

The invention relates to a composition that comprises the peptide QBP1 for use to prevent Post-Traumatic Stress Disorder and related disorders (such as acute stress disorder and general adaptation disorder). The invention can be circumscribed to the Medicine field.

### BACKGROUND ART

The correct development of an individual depends on long-term memory acquisition. Structural changes in individual proteins, and in supramolecular protein assemblies, are proposed to play an important role in both short and long-term memory.

"Amyloids", whose cross-β fold has been postulated as the ancestral protein fold, were initially found to be associated with fatal neurodegenerative disorders. However, more than a century after the identification of these "neurotoxic proteins", there is a growing list of proteins that form the so-called "functional amyloids", which serve a wide variety of physiological functions (Fowler, D.M., et al., Functional amyloid--from bacteria to humans, Trends Biochem Sci (2007) 32 (5): 217-24). Among many functional amyloids, the amyloid formation by neuronal cytoplasmic polyadenylation element binding protein (CPEB) is of great interest because the nervous system is particularly susceptible to amyloid-based diseases, some of which lead to cognitive deficit, such as Alzheimer's, Parkinson's and Hungtinton's diseases. The CPEB family of proteins regulates dormant mRNAs translation and some family members are involved in synaptic plasticity and long-lasting memory. The CPEBs best characterized are those from *Aplysia* (ApCPEB) and *Drosophila* genus (Orb2). In mice an isoform with a glutamine-asparagine (Q/N)-rich terminal region has been related to memory consolidation (mCPEB3) while in humans, although CPEB isoforms have been described, the isoform with a Q/N-rich terminal region that is associated to long-term memory has not yet been identified.

In *Aplysia* genus, CPEB isoforms share a common C-terminal catalytic region (RNA-binding domain), but only the neuronal specific isoform (ApCPEB) has a Q/N-rich N-terminal domain (48.1% Q+N content and predicted to have high conformational flexibility that resembles yeast prion-like domains (PLD)). Indeed, ApCPEB undergoes a conformational transition to a β-sheet-rich state similar to those found in other prion-like proteins. In sensory neurons, the neurotransmitter serotonin controls the prion-like switch between the monomeric inactive form and the self-sustaining prion-like oligomeric state, which is important for the serotonin induced increase in synaptic strength. Based on these results it was postulated that the switch to its oligomeric, self-perpetuating prion-like state contributes to the long-term maintenance of synapse-specific changes, providing a molecular mechanism for the persistence of memory (Si K, Lindquist S, Kandel ER. A neuronal isoform of the Aplysia CPEB has prion-like properties. Cell. 2003;115(7):879-91).

The *Drosophila* orthologue of CPEB, Orb2, has two isoforms structurally similar to the ApCPEB isoform: Orb2A and Orb2B. Both forms are expressed in the fly brain and they are required for long-term memory. One of the Orb2 isoforms, Orb2A, is low-abundant and its availability is controlled by phosphorylation. This isoform has an 8 amino-acid residue segment preceding the N-terminus of the PLD, which is critical for the efficiency as well as the nature of the amyloid-like oligomers formed (Tomita K, et al. Structure-activity relationship study on polyglutamine binding peptide QBP1. Bioorg Med Chem. 2009;17(3):1259-63.). Orb2B, the longer isoform (with a region of 162 residues preceding the PLD), appears to act as a canonical CPEB that regulates local translation via its RNA-binding domain. The PLD containing region of Orb2 (excluding the initial 8 or 162 residues at the N-terminus of Orb2A and Orb2B, respectively) has a low Q/N content in comparison with the ApCPEB (23.5% vs 48.1%), works independently of its RNA-binding domain for long-term memory, and its mutations prevent the consolidation of memory (Keleman K, et al. Function of the Drosophila CPEB protein Orb2 in long-term courtship memory. Nat Neurosci. 2007;10(12):1587-93; Majumdar A et al. Critical role of amyloid-like oligomers of Drosophila Orb2 in the persistence of memory. Cell. 2012;148(3):515-29).

In certain cases there would be useful to prevent the consolidation of memories or even eliminate them once they are consolidated, for example in case of Post-Traumatic Stress Disorder (PTSD). PTSD is recognized as an anxiety disorder which develops following exposure to an extremely traumatic event, it develops after a terrifying ordeal that involved physical harm or the threat of physical harm. People who have PTSD may feel stressed or frightened even when they're no longer in danger. The diagnosis of PTSD is made when the following core symptoms follow exposure to the trauma: the traumatic event is persistently re-experienced (via intrusive thoughts, dreams, flashbacks, or internal and external cues); there is persistent avoidance of evidence associated with the trauma or generalized psychological numbing and isolation; and there is widespread physiologic arousal which was not present prior to the trauma. Nowadays there is not an effective method either to prevent or to eliminate those bad memories associated with PTSD or with other bad experiences.

It has been described a method to erase memory using histone deacetylation inhibitors (Graff, J. et al. 2014. Epigenetic priming of memory updating during reconsolidation to attenuate remote fear memories. Cell 156 (1-2):261-276) but this approach is not useful in blocking memory consolidation.

There is an urgent need to find an efficacious method to help people with PTSD or its related disorders to block memory consolidation after a traumatic stress and not to erase the memory of events that are previous to the traumatic stress (which are the basis of people's identity).

### SUMMARY OF THE INVENTION

The present invention discloses the use of the minimal active core of the peptide QBP1 ("QBP1 fragment" for short from now on, SEQ ID NO: 1) or variants thereof, for use in the prevention or treatment of Post-Traumatic Stress Disorder, Acute Stress Syndrome and/or General Adaptation Syndrome. The results obtained can be extrapolated to the complete QBP1 (SEQ ID NO: 2). In this invention a wide array of *in vitro* (bulk and single-molecule) and *in vivo* (cell culture and animal models) techniques are employed to characterize Orb2 structurally and functionally, demonstrating its prion-like and amyloidogenic properties, and to analyze the behavioral consequences of inhibiting its amyloid-like oligomer formation by the peptide of the present invention. As the expert in the field knows, *Drosophila* is a robust model to analyze memory processes and the results obtained in the present invention can thus be readily extrapolated to other animals, in particular to humans.
A first aspect of the invention refers to a composition that comprises a peptide consisting of an amino acid sequence with at least a 70% of identity with SEQ ID NO: 1 (WKWWPGIF), or consisting of SEQ ID NO: 1 or SEQ ID NO: 2, for use in the prevention or treatment of Post-Traumatic Stress Disorder (PTSD), Acute Stress Syndrome/Disorder (ASD) and/or General Adaptation Syndrome (GAS).
Therefore, the present invention also refers to the use of a peptide consisting of an amino acid sequence with at least a 70% of identity with SEQ ID NO: 1 (WKWWPGIF), or consisting of SEQ ID NO: 1 or SEQ ID NO: 2, for the manufacture of a medicament for the prevention or treatment of PTSD, ASD and/or GAS.

The amino acid sequence described can be of any length, preferably between 2 and 50 amino acids, more preferably between 2 and 30 amino acids, more preferably between 2, 3, 4, 5, 6, 7, 8, 9, 10, 11, 12, 13, 14, 15, 16, 17, 18, 19, 20, 21, 22, 23, 24 or 25 amino acids.
The term "identity", as used herein, refers to the percentage of identical amino acid residues between two peptides when they are compared. The sequence comparison methods are known in the art, and include but are not limited to, the BLASTP or BLASTN, and FASTA program ClustalW. We can consider that peptides identity percentages of at least 70% maintained the same properties of said polypeptide.
The percentage of identity in the present invention is of at least 70%; therefore, it can be 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%.

From now on in the present description the peptide with at least a 70% of identity with SEQ ID NO: 1 will be named as the "peptide of the invention", preferably is SEQ ID NO: 1.
In general, those skilled in the art will appreciate that minor deletions or substitutions may be made to the amino acid sequences of peptides of the present invention without unduly adversely affecting the activity thereof. Thus, peptides containing such deletions or substitutions are a further aspect of the present invention. In peptides containing substitutions or replacements of amino acids, one or more amino acids of a peptide sequence may be replaced by one or more other amino acids wherein such replacement does not affect the function of that sequence. Such changes can be guided by known similarities between amino acids in physical features such as charge density, hydrophobicity/hydrophilicity, size and configuration, so that amino acids are substituted with other amino acids having essentially the same functional properties.

All peptide sequences mentioned herein are written according to the usual convention whereby the N-terminal amino acid is on the left and the C-terminal amino acid is on the right. Once made, changes can be routinely screened to determine their effects on the function of the corresponding peptide by screening procedures known by the expert in the field.

The peptide of the invention can be modified with chemical groups, for example acetyl or amino groups in any of its extremes.

Peptides of the present invention may be made in accordance with techniques known in the art. Using accepted techniques of chemical synthesis, the peptide may be built up either from the N-terminus or, more typically, the C-terminus using either single amino acids or preformed peptides containing two or more amino acid residues.

Particular techniques for synthesizing peptides include classical methods in which peptides of increasing size are isolated before each amino acid or preformed peptide addition; classical chemical synthesis amino acid by amino acid; and solid phase peptide synthesis in which the peptide is built up attached to a resin such as a Merrifield resin. In these synthetic procedures, groups on the amino acids will generally be in a protected form using standard protecting groups such as t-butoxycarbonyl. If necessary, these protecting groups are cleaved once the synthesis is complete. Chemistry synthesis methods for example by peptide synthesis in solid phase, solution synthesis, a combination of methods of solid phase synthesis and solution or enzymatic synthesis, are known by the expert in the field. Peptides of the present invention may also be produced through recombinant DNA procedures as are known in the art. Modifications may be introduced during or after the synthesis of the peptide, for example a label attached for purification, a purification tag.

The term "purification tag" or "affinity tag" as used herein refers to an amino acid sequence which has been incorporated (generally, genetically engineered) to a protein or peptide to facilitate purification (for example in affinity chromatography). Purification tags known in the prior art are for instance, but not limited to, the calmodulin-binding peptide (CBP), the enzyme glutathione-S-transferase (GST) or histidine residues (HIS-tag).

Herein the terms "amino acid sequence", "polypeptide", "peptide" and "oligopeptide" are used interchangeably.

In the present description "inhibition of memory consolidation" is the blocking the long-term establishment of memories. The inhibition is performed preferably before memory consolidation, although it could be done at a later time (using a protocol for memory reconsolidation). Those terms ("memory consolidation" and "long-term establishment of memories") are well known to the expert in the field (see for example, Bailey CH, et al. The persistence of long-term memory: a molecular approach to self-sustaining changes in learning-induced synaptic growth. Neuron. 2004 Sep 30;44(1):49-57).

A preferred embodiment of this description refers to the composition for use in the inhibition of memory consolidation, more preferably wherein the inhibition of memory consolidation is performed after a traumatic event and even more preferably before memory consolidation. Therefore, the peptide of the invention is useful to prevent or treat Post-Traumatic Stress Disorder.

The Post-Traumatic Stress Disease (or Post-traumatic Stress Disorder, PTSD) is a disease developed after a terrifying ordeal that involved physical harm or the threat of physical harm. The term PTSD is a well-known term to the expert in the field (see for example, Vieweg WV, et al. Posttraumatic stress disorder: clinical features, pathophysiology, and treatment. Am J Med. 2006 May;119(5):383-90). Related disorders to PTSD are acute stress disorder and general adaptation syndrome, also known by the expert in the field.

Non-limiting examples of traumatic events (or trauma) include military combat, terrorist incidents, physical assault, sexual assault, motor vehicle accidents, and natural disasters.

Symptoms of PTSD may refer to, for example, but not limited to recurrent and intrusive trauma recollections, recurrent and distressing dreams of the traumatic event, acting or feeling as if the traumatic event were recurring, distress when exposed to trauma reminders, physiological reactivity when exposed to trauma reminders, efforts to avoid thoughts or feelings associated with the trauma, efforts to avoid activities or situations, inability to recall trauma or trauma aspects, markedly diminished interest in significant activities, feelings of detachment or estrangement from others, restricted range of affect, sense of a foreshortened future, social anxiety, anxiety with unfamiliar surroundings, difficulty falling or staying asleep, irritability or outbursts of anger, difficulty concentrating, hyper-vigilance, problems with pain perception, pain tolerance, and exaggerated startle response. In certain embodiments, potentially threatening stimuli can cause hyper-arousal or anxiety. In certain embodiments, the physiological reactivity manifests in at least one of abnormal respiration, abnormal cardiac rate of rhythm, abnormal blood pressure, abnormal function of a special sense, and abnormal function of sensory organ. In certain embodiments, restricted range of effect characterized by diminished or restricted range or intensity of feelings or display of feelings can occur and sense of a foreshortened future can manifest in thinking that one will not have a career, marriage, children, or a normal life span. In certain embodiments, children and adolescents may have symptoms of PTSD such as, for example and without limitation, disorganized or agitated behaviour, repetitive play that expresses aspects of the trauma, frightening dreams which lack recognizable content, and trauma-specific re-enactment. In certain embodiments, a symptom can be stress associated with memory recall.

In certain embodiments PTSD is characterized by three symptom clusters: re- experiencing/intrusion, avoidance/numbing, and hyper-arousal. As used herein, the term "symptom cluster" refers to a set of signs, symptoms, or a set of signs and symptoms, which are grouped together because of their relationship to each other or their simultaneous occurrence.

As used herein, the term "re-experiencing/intrusion" refers to at least one of recurrent and intrusive trauma recollections, recurrent and distressing dreams of the traumatic event, acting or feeling as if the traumatic event were recurring, distress when exposed to trauma reminders, and physiological reactivity when exposed to trauma reminders. In certain embodiments, the physiological reactivity manifests in at least one of abnormal respiration, abnormal cardiac rate of rhythm, abnormal blood pressure, abnormal function of a special sense, and abnormal function of sensory organ.

As used herein, the term "avoidance/numbing" refers to at least one of efforts to avoid thoughts or feelings associated with the trauma, efforts to avoid activities or situations, inability to recall trauma or trauma aspects, markedly diminished interest in significant activities, feelings of detachment or estrangement from others, restricted range of affect, and sense of a foreshortened future. Restricted range of effect characterized by diminished or restricted range or intensity of feelings or display of feelings can occur. A sense of a foreshortened future can manifest in thinking that one will not have a career, marriage, children, or a normal life span. Avoidance/numbing can also manifest in social anxiety and anxiety with unfamiliar surroundings. As used herein, the term "hyper-arousal" refers to at least one of difficulty falling or staying asleep, irritability or outbursts of anger, difficulty concentrating, hyper- vigilance, and exaggerated startle response. Potentially threatening stimuli can cause hyper-arousal or anxiety.

Patients diagnosed with PTSD may feel "on guard", uneasy, and intensely anxious. Depression, anxiety, panic attacks, and bipolar disorder are often associated with PTSD. Alcohol and drug abuse are also common. In certain embodiments, disorders comorbid with PTSD can include for example but without limitation depression, alcohol and drug abuse.

In a particular embodiment the composition prevents at least one sign or symptom of the PTSD in the patient, wherein the sign or symptom is selected from disorganized or agitated behaviour, problems with pain perception and pain tolerance, headache, difficult falling or staying asleep, repetitive play that expresses aspects of the trauma, frightening dreams which lack recognizable content, and trauma-specific re-enactment.

The term "prevention", as understood in the present invention, is to avoid or reduce the consolidation of memories, preferably of memories associated with a traumatic event, more preferably PTSD or at least one of the symptoms of PTSD. The term "treatment" involves erasing memories associated with a traumatic event, preferably with PTSD or at least one symptom of PTSD, by using a protocol of memory reconsolidation (see for example Graff, J. et al. 2014. Epigenetic priming of memory updating during reconsolidation to attenuate remote fear memories. Cell 156 (1-2):261-276). For example, a protocol of memory re-consolidation includes psychotherapy.

The term "treatment" as understood in the present invention, is to cure, alleviate, mitigate, relief, remedy or reduce PTSD or at least one symptom of PTSD.

A second aspect of the present invention refers to the composition that comprises the nucleotide sequence that codifies for the amino acid sequence described in the first aspect of the present invention for use in the prevention or treatment of PTSD, ASD and/or GAS. Therefore, the nucleotide sequence that codifies for the peptide of the invention is useful to prevent or treat PTSD or the related disorders (for example, acute stress disorder or general adaptation disorder). From now on the nucleotide sequence described in the second aspect of the present invention will be named as the "the nucleotide sequence of the invention".

Herein the terms "nucleotide sequence", "polynucleotide", "nucleotide sequence", "nucleic acid" and "oligonucleotide" are used interchangeably.

The polynucleotides of the present invention may have conservative substitutions, known to the skilled expert. Conservative substitutions lead to the polynucleotides that encode the same peptide.

The term polynucleotide includes genomic DNA or DNA encoding double or single stranded, ribonucleic acid (RNA), any synthetic and genetically manipulated polynucleotide, and both sense and antisense strands. This includes single-chain molecules and double-stranded, such as DNA-DNA hybrids, DNA-RNA and RNA-RNA.

The nucleotide sequence of the invention can be obtained artificially by conventional cloning methods selection and widely known in the prior art.

The nucleotide sequence of the invention, in addition to the coding sequence, it may have other elements, such as but not limited, introns, noncoding sequences at the 5 'or 3', binding sites for ribosomes, stabilizing sequences, cell detection, etc. These polynucleotides may also include further coding sequences for additional amino acids which may be useful, for example, but not limited, to enhance stability of the peptide generated from it or to allow a better purification thereof.

The nucleic acid can form part of an expression cassette, comprising the nucleic acid of the invention operably linked to control elements of transcription and/or translation.

The polynucleotide sequences described in the second aspect of the present invention may be found forming part of vectors allowing the propagation or cloning and expression. Said vector may be, for example a cloning vector or an expression vector or recombinant vector. The cloning of the nucleotide sequence of the invention may be performed using an expression vector or recombinant or plasmid. These vectors comprise the nucleotide sequence of the invention (or the expression cassette comprises) and may also comprise a series of amino acid coding sequences and targets for protease cleavage. The advantage of this structure is that once produced the fusion protein or peptide, lysate expression system and purified it by, for example, but not limited to, affinity chromatography, may be cleaved the peptide from the carrier protein of the invention by digestion with a protease and repurification the peptide of the invention using the same chromatographic system.

The term "vector", as used herein, refers to a nucleic acid molecule that is capable of transferring nucleic acid sequences contained therein to a cell. Examples of recombinant vectors are linear DNA, plasmid DNA, modified viruses, adenovirus/adeno-associated viruses, and retroviral viral vectors, etc.; all widely described in the literature and which can be used following standard molecular biology techniques or purchased from vendors. Among the most commonly used are viral vectors for the introduction of genes in mammalian cells such as poxvirus vectors, herpes simplex, adenovirus, adeno-associated vectors, etc.

Vectors may be introduced by any method known to the skilled artisan, for example, but without limiting, by transfection, transformation or infection of the host cells, such as, but not limited to, plant cells, mammalian, bacteria, yeast or insect cells.

The term "cloning vector" as used herein, refers to a DNA molecule in which one can integrate a DNA fragment, without loss of replication capacity. Examples of expression vectors are, without limitation, plasmids, cosmids, phage DNA or yeast artificial chromosomes.

The term "expression vector", as used herein, refers to a suitable cloning vector to express a nucleic acid that has been cloned therein after being introduced into a cell, called host cell. Said nucleic acid is generally operably linked to control sequences.

The term "expression" refers to the process by which a polypeptide is synthesized from a polynucleotide. Includes transcription of the polynucleotide in a messenger RNA (mRNA) and the translation of such mRNA into a protein or polypeptide. Expression may take place in a host cell.

The term "recombinant vector", as used in the present description refers to a vector suitable for expressing a nucleic acid that has been cloned therein, so that the expression of the peptide is directly in the tissue or target cell. Generally said vector is a virus, and is produced by the binding of different nucleic acid fragments from different sources and whose expression results in an infective viral particle composed typically of coat protein, viral genome and proteins associated with viral genome. Expression in a tissue or cell of interest is accomplished by operably linking the polynucleotide to control sequences, specific control sequences preferably the tissue or cell where it is meant; preferably such control sequences are enhancers or promoters. A typical recombinant vector is selected from the group consisting of a lentiviral vector, an adenoviral vector and / or an adeno-associated virus vector.

A preferred embodiment of the second aspect of the invention relates to the composition for use wherein the nucleotide sequence is comprised in a vector selected from the list that consists of: plasmid, bacmid, yeast artificial chromosome, bacteria artificial chromosome, bacteriophage, cosmid, adenovirus, retrovirus, lentivirus or any combinations thereof.

In the present invention the terms "composition", "pharmaceutical composition", "drug" and "medicament" are used interchangeably.

The term "pharmaceutical composition" herein refers to any substance used for prevention, diagnosis, alleviation, treatment or cure of diseases in humans or animals. The pharmaceutical composition of the invention can be used either alone or in combination with other pharmaceutical compositions. The term pharmaceutical composition and drug are used in this invention interchangeably. In the context of the present invention relates to a pharmaceutical composition or medicament characterized by comprising the peptide of the invention or the polynucleotide that codifies it allowing their expression in the organism to be treated in a therapeutically effective amount, such that the peptide of the invention performs its function in the target tissue or cell.

In a preferred embodiment of the first and second aspect the composition for use also comprises an excipient and/or an acceptable pharmaceutic carrier.

The term "excipient" refers to a substance that helps absorption of the elements of the composition of the invention and actively stabilizes or assists the preparation of the composition in the sense of giving consistency or flavour. Thus, carriers may have the function of holding the ingredients together, such as in the case of starches, sugars or celluloses, sweetening function, the function as a colorant, the protective function of the composition, such as to isolate the air and / or moisture, filling the role of a tablet, capsule or other form of presentation, such as is the case of dibasic calcium phosphate, the disintegrating function to facilitate dissolution of the components and its absorption in the intestine, without excluding other excipients not mentioned in this paragraph.

The term "pharmaceutic carrier", refers to a substance used in the pharmaceutical composition or medicament to dilute any component of the present invention included therein to a given volume or weight. The vehicle function is to facilitate the incorporation of other elements, allow better dosage and administration or give substance and form to the composition. When the presentation is liquid, the pharmacologically acceptable carrier is the diluent.

In another preferred embodiment of the first and second aspect the medicament for use also comprises an adjuvant.

Herein, the term "adjuvant" refers to an agent that increases the effect of the peptide of the invention when given jointly to it or forming part of the same treatment protocol. The pharmaceutically acceptable adjuvants and vehicles that may be used in the pharmaceutical composition of the present invention are vehicles known by those skilled in the art.

In another preferred embodiment of the first and second aspect the medicament also comprises another active principle, like existing drugs to treat PTSD (anti-anxiety medications, antidepressants, prazosin, etc.).

As used herein, the term "active principle" ("active substance", "pharmaceutically active substance", "active ingredient" or "pharmaceutically active ingredient") means any component that provides pharmacological activity potentially or a different effect in the diagnosis, cure, mitigation, treatment, or prevention of disease, or to affect the structure or function of the body of man or other animals.

Antidepressants are medications that can help symptoms of depression and anxiety. They can also help improving sleep problems and concentration. For example, the selective serotonin reuptake inhibitor (SSRI) medications sertraline (Zoloft®) and paroxetine (Paxil®) are approved by the Food and Drug Administration (FDA) for PTSD treatment.

In the case of anti-anxiety medications, these drugs also can improve feelings of anxiety and stress for a short time to relieve severe anxiety and related problems. Because these medications have the potential for abuse, they are not usually taken long term.

If symptoms include insomnia or recurrent nightmares, a drug called prazosin (Minipress®) may help. Although not specifically FDA-approved for PTSD treatment, prazosin may reduce or suppress nightmares in many people with PTSD.

In a more preferred embodiment of the first and second aspect the composition for use additionally comprises an internalization agent selected from the list consisting of: cyclodextrin, poloxamers, receptor binding peptides of acetylcholine, viruses, VLPs (Virus Like Particles), Cereport® (RMP-7), micelles, liposomes, polietilenglicolades, PEG-PLA, PEG-PCL, PEG-PHDCA, TAT 47-57 (SEQ ID NO: 3), TAT 48-60 (SEQ ID NO: 4), Antp (penetratin, SEQ ID NO: 5) or Pep-1 (SEQ ID NO: 6).

The term "internalization agent" refers to a molecule capable of facilitating the passage of a given peptide through the blood-brain barrier (BBB) and the plasma membrane to permit access to the interior of neurons and/or glial cells. This molecule can be covalently bound or not to the peptide of the present invention. Non-limiting examples of the internalization agent can be: cyclodextrin, poloxamers, receptor binding peptides of acetylcholine, viruses, VLPs (Virus Like Particles) Cereport® (RMP-7), micelles, liposomes, nanocarriers (including polyetilenglicolades, PEG-PLA PEG-PCL, PEG-PHDCA, etc) or cell penetrating peptides (CPP) like TAT (for example TAT 47-57, SEQ ID NO: 3; or TAT 48-60, SEQ ID NO: 4), Antp (penetratin, SEQ ID NO: 5) or Pep-1 (SEQ ID NO: 6).

Herein "CPPs" or "cell penetrating peptide" refers to a group of short peptides, of less than 30 amino acids, that are able to penetrate the membrane together with the load carrying up inside the cell without producing cytolytic effects; they are peptides with positive charge, amphipathic characteristics, theoretical hydrophobicity, helicoidal moment, or able to interact with lipid membranes and adopt a distinct secondary structure after its association with lipids. CPPs enter the cell primarily by endocytosis or mediated macropinnocitosis lipid rafts-mediated. CPPs are used as transport bioactive molecules introduced into cells; typically by a covalent linkage, although non-covalent bonds are also possible.

In another particular embodiment, said pharmaceutical composition for use is prepared in solid form or aqueous suspension, in a pharmaceutically acceptable diluent.

The therapeutic composition for use provided by this invention can be administered by any suitable administration route, for which said composition is formulated in the suitable to the chosen route of administration pharmaceutical form.

In a more preferred embodiment of the first and second aspect the medicament for use is presented in an adapted form for oral, parenteral, intramuscular, intraarterial, intravenous, subcutaneous, epidural, intradermic, intraperitoneal or intravesicular administration.
It has been already demonstrated that QBP1 can inhibit amyloidogenesis of other amyloidogenic protein (polyQ tracks) in *Drosophila* when administered orally (Nagai, Y., et al., Prevention of polyglutamine oligomerization and neurodegeneration by the peptide inhibitor QBP1 in Drosophila, Hum Mol Genet (2003) 12 (11): 1253-9).

In a more preferred embodiment of the first and second aspect the medicament for use is administered during 1 to 7 days, preferably between 1 or 2 days, after the trauma.

In a more preferred embodiment of the first and second aspect the medicament for use is administered in a therapeutically effective dose.

In the present invention the term "therapeutically effective dose" (or therapeutically effective amount") refers to the amount of the agent or compound capable of producing a desired effect (in the present invention the prevention or treatment of PTSD, ASD and/or GAS) and will generally be determined by the compounds' characteristics, the route and frequency of administration form thereof and other factors, including age, condition of the patient and the severity of the alteration or disorder.

In a more preferred embodiment of the first and second aspect the prevention or treatment is performed in a human being, man or woman of any age.

Non-human vertebrate animals (Veterinary use) can also be subject to the prevention or treatment. For example horses and dogs are often used in military or police operations as well as in other instances, such as on racetracks, where they may become exposed to traumatic conditions. The therapy of the invention can therefore be of use to restore performance in such animals.

In a more preferred embodiment of the first and second aspect the composition is used not as a prophylactic but as a therapeutic agent to selectively erase memories after the development of PTSD (after the trauma), taking advantage of the phenomenon of memory re-consolidation in a human being (using physoctherapy as a coadjuvant). "Memory reconsolidation", a known term to the expert in the field, is a phenomenon of recalling memories and editing them. In humans, the protocol of memory re-consolidation can be done with psychotherapy as a co-adjuvant.
Blocking memory consolidation by QBP1 could be a potential therapeutic strategy to prevent or treat Post Traumatic Stress Disorder (PTSD) or other memory-related diseases (acute stress disorder, general adaptation syndrome).
The present description also refers to a method for the inhibition of memory consolidation, therefore, for the prevention or treatment of PTSD or other memory-related diseases (acute stress disorder or general adaptation syndrome), that comprises the administration of a therapeutically dose of a composition that comprises an amino acid sequence that comprises a peptide with at least a 70% of identity with SEQ ID NO: 1, preferably in a human. Preferably the sequence comprises the peptide according to SEQ ID NO: 1, more preferably the sequence consists of the peptide according to SEQ ID NO: 1. In another preferred embodiment the composition comprises a peptide consisting of SEQ ID NO: 2; more preferably, comprises the peptide SEQ ID NO: 2; and in a preferred embodiment the amino acid is SEQ ID NO: 2. This method can also comprise the administration of another active principle, like existing drugs to treat PTSD (anti-anxiety medications, such as Zoloft® and paroxetine (Paxil®); antidepressants, for example the selective serotonin reuptake inhibitor (SSRI) medications sertraline (Zoloft®) and paroxetine (Paxil®); or prazosin, (Minipress®)). In this method it can be also used the nucleotide sequence that codifies for the amino acid sequence of the peptide of the invention. In this method it can be also used an internalization agent for the delivery of the peptide or nucleotide of the invention. In this method, the peptide of the invention can be administered via oral, parental, intramuscular, intraperitoneal, intraarterial, intravenous, subcutaneous, epidural, intradermic, intraperitoneal or intravesicular. The composition can be administered during 1-7 days, preferably between 1-2 days after the trauma. This method can also comprise the administration of a protocol of memory reconsolidation, for example using psychotherapy. Treatment may retard progression, prevent, prevent relapse, or ameliorate PTSD, or one or more symptoms of the disorder.
Unless otherwise defined, all technical and scientific terms used herein have the same meaning as commonly understood by one of ordinary skilled in the art to which this invention belongs. Throughout the description and claims the word "comprise" and its variations are not intended to exclude other technical features, additives, components, or steps. The following examples, drawings and sequence listing are provided by way of illustration and are not intended to be limiting of the present invention.

### BRIEF DESCRIPTION OF THE DRAWINGS

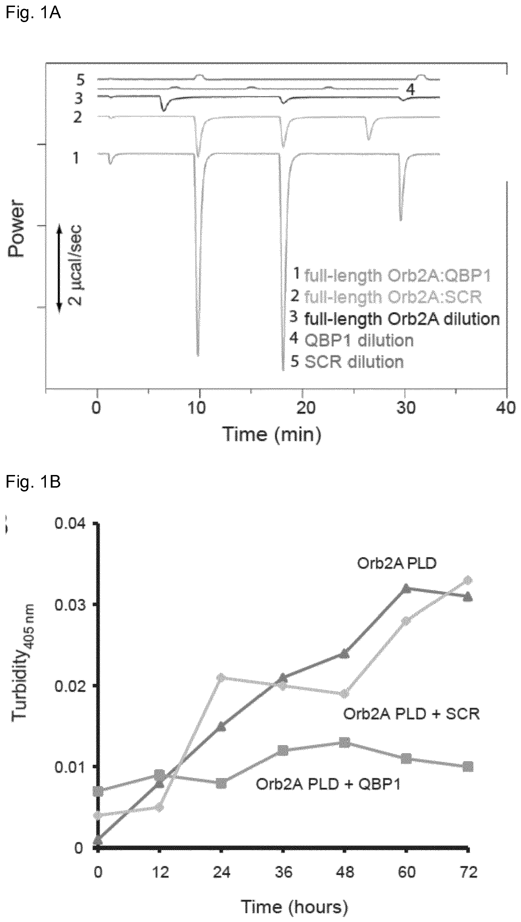

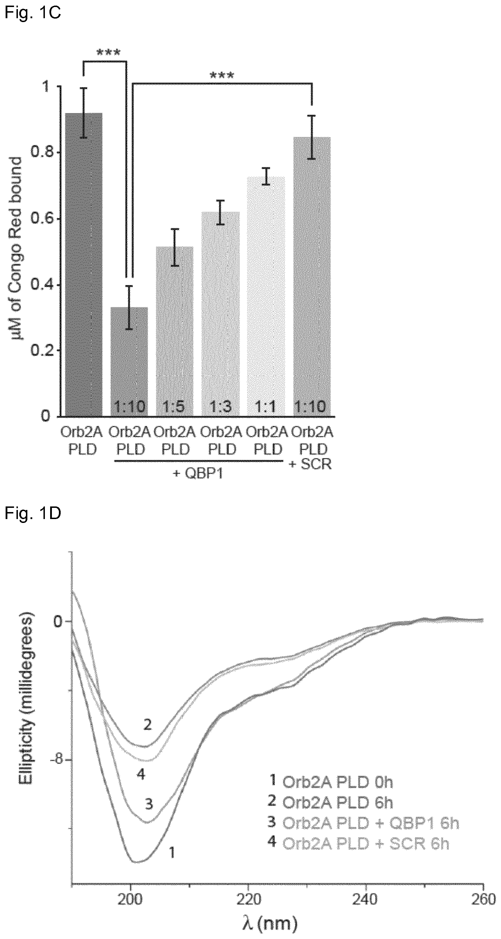

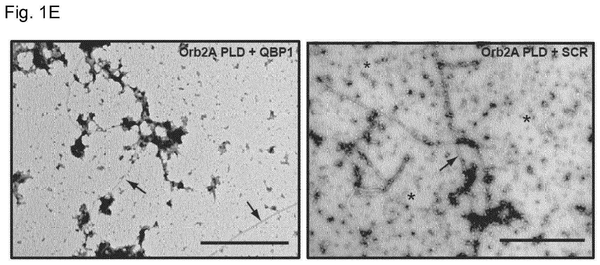

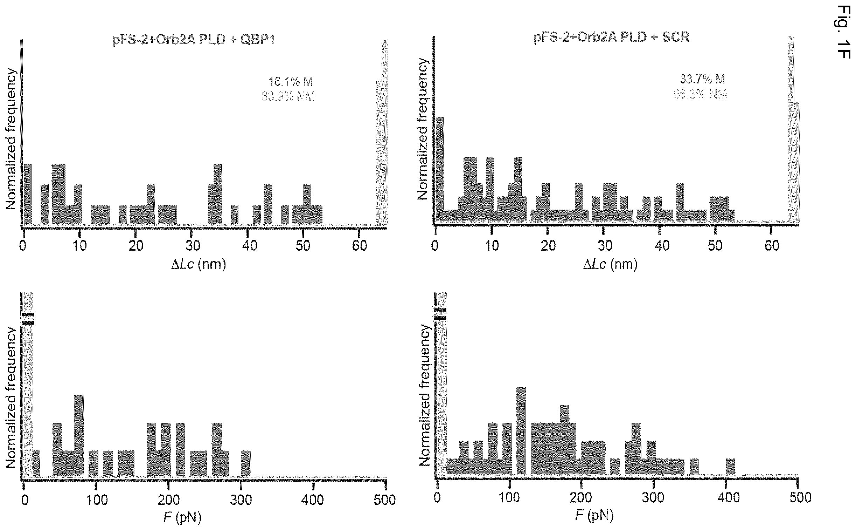

**Fig. 1****. QBP1 fragment (QBP1-f) interferes with Orb2 amyloid formation *in vitro.* (A)** Representative calorimetric traces for the interaction of full-length Orb2A with the QBP1-f and SCR peptides. Traces 1 and 2 correspond to the heat released upon injection of Orb2A into the ITC cell loaded with an excess of QBP1-f or SCR, respectively, while traces 3, 4 and 5 correspond to the Orb2A, QBP1 and SCR dilutions, respectively. **(B)** QBP1-f but not SCR drastically reduced the turbidity at 405 nm of aged Orb2A PLD. **(C)** QBP1-f reduces the quantity of amyloid formed as shown by measuring the CR bound to Orb2A PLD. The data are represented as the mean ± SEM: **p* < 0.05 and ****p* < 0.001 (One-way ANOVA and Tukey post test). **(D)** Far-UV CD spectroscopy in the absence or presence of QBP1 or SCR indicates that QBP1 blocks protein precipitation over time of Orb2A PLD, but SCR does not. **(E)** Representative electron micrographs show that oligomers (asterisks) and amyloid fibers (arrows) of Orb2A PLD were drastically reduced when incubated with QBP1 (left) but not with the SCR (right). Scale bars: 1 *µ*m. **(F)** The conformational polymorphism of Orb2A PLD is strongly diminished in the presence of a 1:10 molar excess of QBP1-f, which decreases the M frequency relative to the NM conformers (QBP1-f, n=112 and SCR, n=101). (QBP1: QBP1-f). ΔLc: increase in contour length. M: mechanoestable events, NM: on mechanostable events.

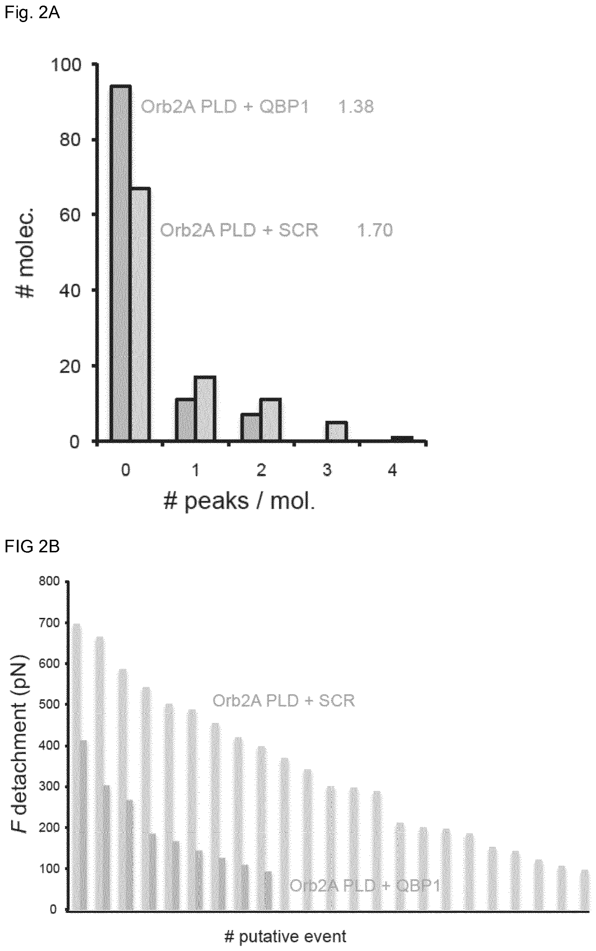

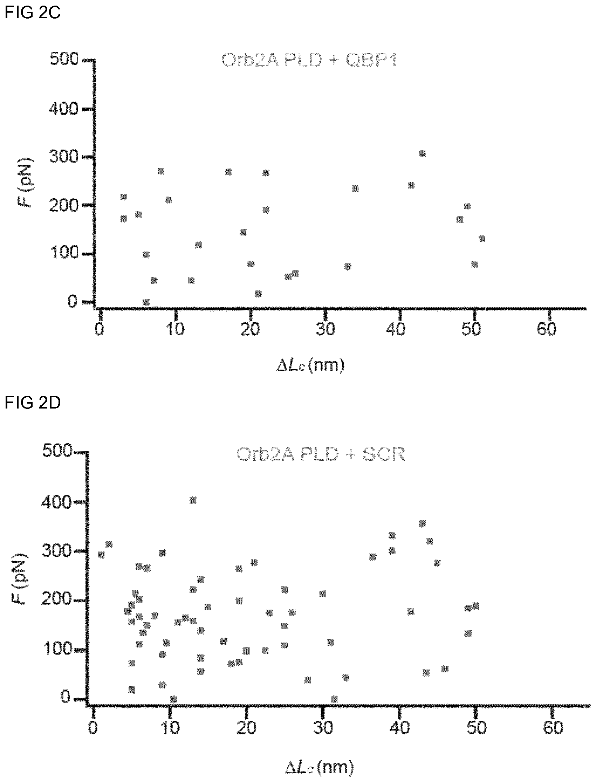

**Fig. 2****. Additional AFM-SMFS analysis of Orb2A PLD in the presence of QBP1-f**
   **(A)** The QBP1-f peptide reduces the number of force peaks *per* molecule, suggesting that it decreases the propensity of Orb2A PLD to adopt *β-*structures (numbers in the inset are the average number of force peaks per Orb2A molecule). **(B)** Incomplete recordings of putative M events decrease in the presence of QBP1 inhibitor peptide, which suggests the formation of fewer, and weaker, M conformers. **(C and D)** No preferential structures were detected when stretching pFS-2+Orb2A PLD in the presence of QBP1-f ((C) or the SCR (D). (QBP1: QBP1-f).

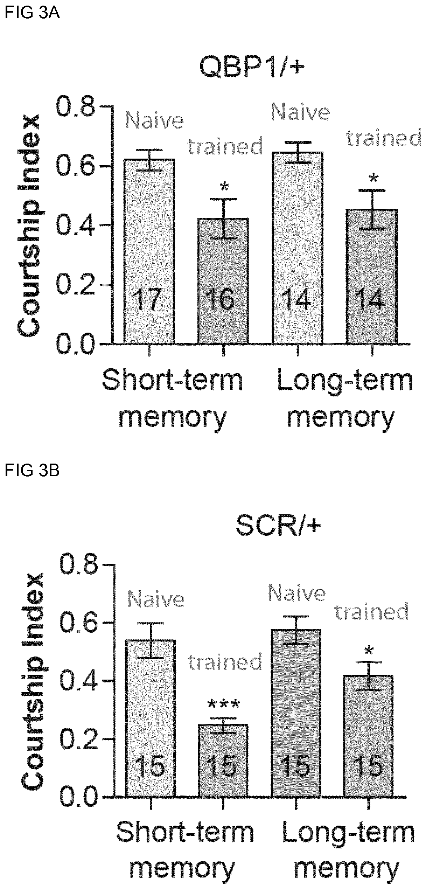

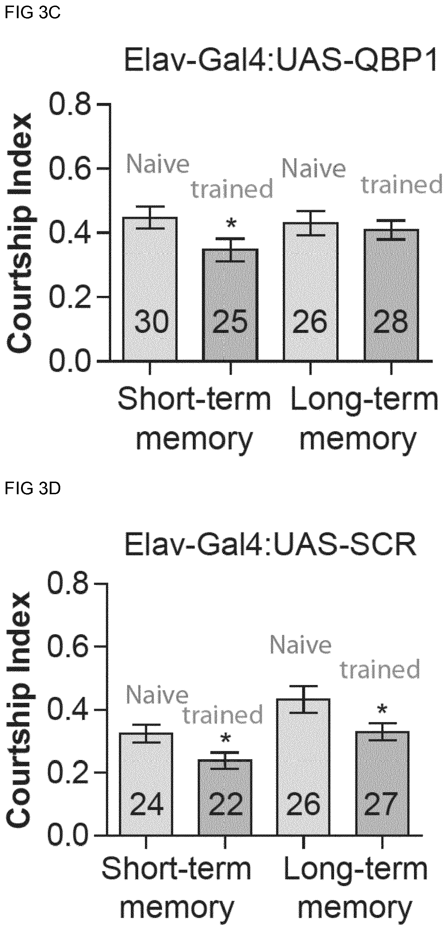

**Fig.3****. Expression of QBP1-f interferes with long-term male courtship suppression**
   Courtship index of male flies that were exposed to an unreceptive female was compared to that of naïve, untrained male flies (A,B). Pan-neuronal expression of QBP1-f using the Elav-Gal4 driver interferes with long-term but not short-term memory (C),, while the SCR control peptide has no effect (D). The genetic controls for each group were tested simultaneously and the number of flies used in each group is indicated. (QBP1: QBP1-f). QBP1/+: heterozygous QBP1; SCR/+: heterozygous SCR (scrambled). UAS-QBP1: module carrying QBP1; UAS-SCR: module carrying SCR.

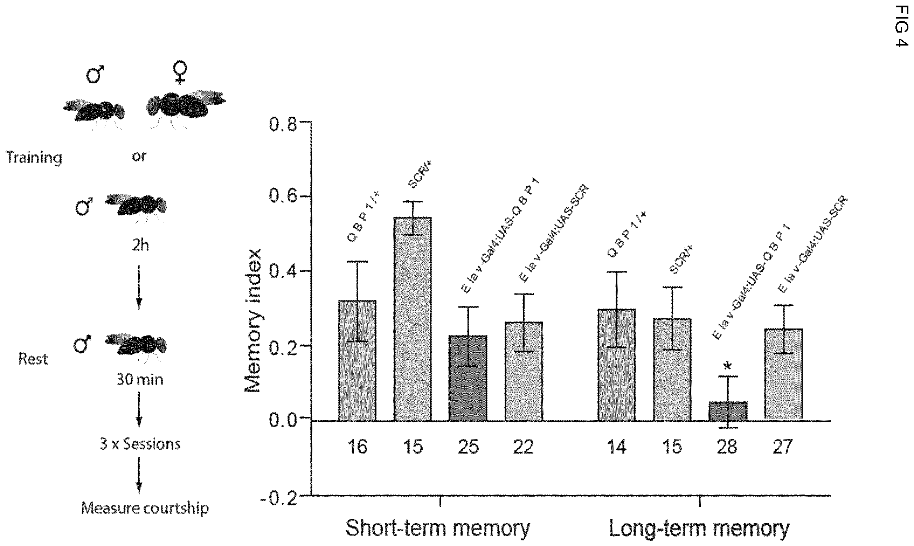

**Fig.4****. QBP1-f interferes with Orb2-mediated memory consolidation *in vivo.*** Pan-neuronal expression of QBP1-f disrupts long-term male courtship suppression memory, while it does not interfere with short-term memory. The data are represented as the mean ± SEM. We assumed statistical significance at **p* < 0.05 (One-way ANOVA). n= 16, 15, 25, 22, 14, 15, 28, 27 from left to right. (QBP1: QBP1-f).

### EXAMPLES

### Example 1. QBP1 blocks amyloidogenesis and memory consolidation

The conformational similarity but distinct cellular outcome prompted the inventors to further examine the conformational landscape of Orb2 with additional tools. Since in a previous work (Hervas R, et al. Common features at the start of the neurodegeneration cascade. PLoS Biol. 2012;10(5):e1001335) we found the polyglutamine binding peptide 1, QBP1 (a known inhibitor of amyloids including HttQ expansions) was effective also to block the amyloidogenesis of some amyloidogenic proteins, including α-synuclein (causal protein for Parkinson) and Sup35NM (a model yeast prion), but not others (e.g., β-amyloid) we wonder to which class Orb2/CPEB would belong. In addition to these uncertainties, it was not at all obvious that an anti-amyloidogenic agent should inhibit Orb2 amyloidogenesis and, in turn, memory consolidation as previous attempts to do so with other anti-amyloidogenic agents have failed (Kausik Si, known expert in the present field, personal communication). Indeed, this peptide was thought to be rather specific for expanded polyQ tracts (as its own name indicates). Thus here Orb2 was further analyzed with QBP1 for its possible anti-amyloidogenic effect.

QBP1 is a hydrophobic peptide, identified by its ability to bind monomeric expanded polyQ proteins that blocks the critical β-conformational transition of this species, suppressing oligomerization, fibrillogenesis as well as associated cytotoxicity and neurodegeneration (Nagai, Y., et al., A toxic monomeric conformer of the polyglutamine protein, Nature structural & molecular biology (2007) 14 (4): 332-40, Popiel, H.A., et al., Protein transduction domain-mediated delivery of QBP1 suppresses polyglutamine-induced neurodegeneration in vivo, Mol Ther (2007) 15 (2): 303-9,), QBP1 also inhibits amyloidogenesis (conformational polymorphism, oligomerization, fibrillogenesis) in α-synuclein and Sup35 but not in β-amyloid (Hervas, R., et al., Common features at the start of the neurodegeneration cascade, PLoS biology (2012) 10 (5): e1001335) or Tau (Fernández-Ramírez & Carrion-Vázquez, personal communication). Thus, QBP1 acts as a polyvalent anti-amyloidogenic agent on several amyloids (but not all of them, and there is no way in the state of the art to predict this behaviour for a new amyloid) and so, the potential inhibitory effect of the minimal active core of QBP1 (or QBP1 fragment, or QBP1-f), Ac-WKWWPGIF-NH2 (SEQ ID NO: 1) (Tomita, K., et al., Structure-activity relationship study on polyglutamine binding peptide QBP1, Bioorg Med Chem (2009) 17 (3): 1259-63), was examined on Orb2 amyloid formation. The results obtained here with the QBP1-f can be extrapolated to QBP1 and to CPEBs (Orb2 orthologs) from mammals like humans. They also bring direct evidence on the mechanistic relationship between Orb2 amyloidogenesis and memory consolidation.

Isothermal titration calorimetry (ITC) revealed that QBP1-f physically interacts with Orb2A, and the complex formation, a slow event, was exothermic in nature (Fig. 1a). Under similar conditions a scrambled version of QBP1-t, SEQ ID NO: 7 (SCR, Ac-WPIWKGWF-NH₂,), showed marginal interaction, thus serving as a negative control in subsequent experiments (Fig. 1a). Incubation with QBP1-f inhibited Orb2A PLD amyloidogenesis whereas the SCR did not, as monitored by turbidimetry and CR biding (Fig. 1b,c). Far-UV CD analysis revealed that QBP1-f, but not SCR, suppressed the oligomerization processes and decreased the loss of signal due to aggregation and precipitation (Fig. 1d). Furthermore, TEM (Transmission Electron Microscopy) showed a dramatic reduction in the formation of both oligomers and fibers in presence of QBP1-f but not SCR (Fig. 1e). Finally, double-blind analysis of SMFS experiments showed a drastic reduction in the formation of M conformers (from 37.7% to 16.1%), shifting the NM/M equilibrium by QBP1-f towards non-mechanostable conformers, while SCR had no effect on this conformational transition (Fig. 1f). Interestingly, with QBP1-f there were fewer structures formed per molecule (Fig. 2a) and fewer putative M events (Fig. 2b), the latter showing a similar decrease than with bona fide (identified) M events. The lack of association between ΔLc and F was also found in the presence of QBP1-f (Fig. 2c) indicating that no preferred structure forms. Taken together, these results indicate that QBP1-f interferes *in vitro* with Orb2 amyloid formation and suggests that Orb2 and pathological amyloid forming polypeptides adopt also similar conformers early in amyloidogenesis that are recognized and blocked by QBP1-f.

Since QBP1-f inhibits the transition of monomeric Orb2A to attain the conformations leading to amyloid formation, it was next studied the physiological consequences of QBP1 on memory formation in *Drosophila melanogaster.* To this end, it was used the male courtship suppression memory as a functional assay. A virgin male repeatedly exposed to unreceptive female learns to suppress courtship towards the unreceptive females. Expression of QBP1-f or the control SCR in the nervous system did not affect fly development although the normal courtship behaviour was slightly diminished (Fig. 3). Following training, flies expressing the peptide (Elav-Gal4:UAS-QBP1 or SCR) and the genetic controls (Elav-Gal4 and UAS-QBP1 or UAS-SCR) (Brand AH, Perrimon N. Targeted gene expression as a means of altering cell fates and generating dominant phenotypes. Development. 1993;118(2):401-15) had similar courtship suppression immediately after training, suggesting that the expression of the peptides does not interfere with short-term memory. However, when measured after 24 hours, the Elav-Gal4:UAS-QBP1 males displayed elevated courtship, which indicates loss of long-term memory, compared to the control Elav-Gal4: UAS-SCR flies (Fig. 4).

This set of results suggests that initially Orb2A amyloid formation follows a pathway similar to that of pathological amyloids, and that the anti-amyloidogenic reagents such as QBP1 peptide can interfere with memory processes.

Similar *in vitro* experiments with QBP1 have been done for the ApCPEB with similar results (Hervás & Carrión-Vázquez, personal communication). This makes our conclusions more general for the Orb2/CPEB family, further supporting our hypothesis that QBP1 will also inhibit memory consolidation in mammals (mice and humans), mostly considering the high divergence existing between Orb2 and ApCPEB, which is comparable to that between them and those from mammals.

Taking in count that the percentage of identity among those species are the following: ApCPEB and Orb2A: 28.73%; Orb2A and mouse CPEB3 58.92%; and mouse CPEB3 and human CPEB 97.84%; the results obtained in the present invention can be extrapolated to humans.

### Methods summary

Detailed information regarding AFM-SMFS, CD, turbidimetry, CR binding assay, TEM experiments, can be found in (Hervas, R., et al., Common features at the start of the neurodegeneration cascade, PLoS biology (2012) 10 (5): e1001335). Details on polyprotein sample preparation, using a new vector recently described by our group (pFS-2) can be found in (Hervas, R., et al., Common features at the start of the neurodegeneration cascade, PLoS biology (2012) 10 (5): e1001335, Oroz, J., et al., Unequivocal single-molecule force spectroscopy of proteins by AFM using pFS vectors, Biophys J (2012) 102 (3): 682-90).

Buffers, time and storage conditions, and protein/peptide concentrations used in the aforementioned techniques were as follows:
- AFM-SMFS: Experiments were performed using 2-3 µM polyproteins in 10 mM Tris-HCI pH 7.5 at room temperature with proteins incubated at 4ºC between sessions and NTA-Ni2+ functionalized coverslips. For experiments in the presence of the peptides QBP1 and SCR, 20 µM of the peptide were used dissolved in DMSO, and the samples were incubated overnight with the peptide at 4ºC before the measurement.
- CD: For far-UV CD spectra it was used a protein concentration of 2-5 µM in 5 mM NaH2PO4, with 0.02 % NaN3, pH 6.0. The QBP1 or SCR (when used) was five fold molar excess relative to proteins (final concentrations 10 to 25 µM with the final concentration (v/v) of DMSO below 0.01%). After recording the first protein spectrum (hour 0), the protein was incubated at 37ºC without stirring and additional spectra were collected at the indicated times. The secondary structure content of each protein and its evolution during incubation were estimated by spectra deconvolution using the CDNN analysis program (Bohm, G., et al., Quantitative analysis of protein far UV circular dichroism spectra by neural networks, Protein Eng (1992) 5 (3): 191-5). For thermal stability measurements, the ellipticity changes induced by increasing the temperature (at a constant heating rate of 60ºC/h) were measured at 222 nm in 5 mM NaH2PO4 pH 6.0 using a protein concentration of 20 µM.
- Turbidimetry: Protein samples used were at 10 µM in PBS+0.02% NaN3, pH 7.0 for all the kinetic experiments. Samples were incubated at 37ºC without stirring. SCR and QBP1 peptides were used at 50 µM.
- CR binding assay: Samples were aged for 7 days at 37ºC with 0.02% NaN3 and no stirring prior to performing the measurements. The protein samples (10 µM) were dialyzed in PBS pH 7.0 for the experiments. SCR and QBP1 peptides were used at 50 µM.
- TEM imaging: Protein samples (10 µM) were dialyzed in PBS pH 7.0 for all the TEM measurements. Images were taken after 7 days of incubation at 37ºC in the presence of 0.02% NaN3 without stirring. SCR and QBP1 peptides were used at 50 µM.
- ITC: ITC experiments of full-length Orb2A interaction with the minimal active core of QBP1 and SCR peptides (Tomita, K., et al., Structure-activity relationship study on polyglutamine binding peptide QBP1, Bioorg Med Chem (2009) 17 (3): 1259-63) were carried out in a VP-ITC microcalorimeter at 25ºC. Proteins were equilibrated in PBS, pH 7.4, by gel-filtration chromatography and the equilibration buffer was used to prepare the peptide solutions. Protein/peptide binding was tested by successive injections of protein sample (10 to 25 µl each) into the reaction cell loaded with peptide at high final [peptide]/[protein] molar ratios. The apparent heat of reaction for each injection was obtained by integration of the peak area. The heats developed on protein or peptide dilutions were determined in separate runs loading the sample cell or the injection syringe with buffer, respectively, in the conditions used for the binding experiments. The complexity of the system and the lack of precise information on the distribution of Orb2A conformations before and after complex formation precluded the quantitative analysis of titration curves. Protein (Orb2A, 185 µM) and ligand (QBP1 and SCR, 300 µM and 51 µM, respectively) concentrations in the loading solutions were measured spectrophotometrically using their respective extinction coefficients.
- *Drosophila* strains. It was used the following Drosophila strains from Bloomington Stock Center: Elav-Gal4 (stock no. 458), Drl-Gal4 (stock no.4669), and UAS-GFP (stock no. 1522). The following transgenic lines were generated by us for the research described here: UAS-Orb2A-EGFP, UAS-Orb2B-EGFP. Various genetic combinations were made by standard genetic crosses. It was used the UAS-QBP1 and UAS-SCR lines. The expression level of QPB1 and SCR was similar (Nagai, Y., et al., Prevention of polyglutamine oligomerization and neurodegeneration by the peptide inhibitor QBP1 in Drosophila, Hum Mol Genet (2003) 12 (11): 1253-9).

For behavioural assays flies were maintained using standard fly husbandry methods. Briefly, flies were raised on standard cornmeal food at 25°C and 60% relative humidity on a 12h/12h light:dark cycle. All flies (1 day old) were collected and placed in a vial with fresh food for 24 h at 25°C prior to behavioural testing and all transgenic lines were backcrossed at least 6 times with white eyed Canton-S flies. All controls and transgenic lines carried one copy of the mini white gene known to influence fly behaviour.

Male courtship suppression assay. The male courtship conditioning was modified from McBride and colleagues (McBride, S.M., et al., Mushroom body ablation impairs short-term memory and long-term memory of courtship conditioning in Drosophila melanogaster, Neuron (1999) 24 (4): 967-77). A five-day-old male virgin was paired with a freshly mated female for 3 sessions. Each session lasted for 2 h, with 30 min rest in between. Memory performance was tested with a fresh-mated female at 5 min and 24 h time intervals after the three sessions of spaced training. Courtship Index (CI) was measured as the fraction of time the tested male spent on chasing the female in 10 min time intervals. Memory Index was calculated using CI Naive and CI Trained, which are the mean courtship indices for independent samples of naive and trained males, respectively.

GraphPad Prism version 4.0 was used for statistical analysis. A statistical significance at *p < 0.05 was assumed. One-way ANOVA was used for comparing memory index between each genotype.

## Claims

1. A composition that comprises a peptide consisting of an amino acid sequence with at least a 70% of identity with SEQ ID NO: 1, or consisting of SEQ ID NO: 1 or SEQ ID NO: 2, for use in the prevention or treatment of Post-Traumatic Stress Disorder, acute stress syndrome and/or general adaptation syndrome.

2. A composition that comprises the nucleotide sequence that codifies for the amino acid sequence described in claim 1 for use in the prevention or treatment of Post-Traumatic Stress Disorder, acute stress syndrome and/or general adaptation syndrome.

3. The composition for use according to any one of claims 1 or 2 that additionally comprises an excipient and/or an acceptable pharmaceutical carrier and/or an adjuvant, or any combinations thereof.

4. The composition for use according to any one of claims 1 to 3 that additionally comprises an internalization agent selected from the list consisting of: cyclodextrin, poloxamers, receptor binding peptides of acetylcholine, viruses, VLPs (Virus Like Particles), Cereport® (RMP-7), micelles, liposomes, polietilenglicolades, PEG-PLA, PEG-PCL, PEG-PHDCA, TAT 47-57 (SEQ ID NO: 3), TAT 48-60 (SEQ ID NO: 4), Antp (penetratin, SEQ ID NO: 5) or Pep-1 (SEQ ID NO: 6).

5. The composition for use according to any one of claims 1 to 4 wherein it is presented in an adapted form for oral, parenteral, intramuscular, intraarterial, intravenous, subcutaneous, epidural, intradermic intraperitoneal or intravesicular administration.

6. The composition for use according to any one of claims 1 to 5 wherein the composition is administered during 1 to 7 days, preferably between 1 to 2 days, preferably after the trauma.

7. The composition for use according to any one of claims 1 to 6 wherein the prevention or treatment is performed in a human being.

8. The composition for use according to any one of claims 1 to 7 wherein the prevention is performed before memory consolidation.

9. The composition for use according to any one of claims 1 to 8 wherein the treatment is performed after memory consolidation using a protocol of memory re-consolidation.

## Patentansprüche

1. Zusammensetzung, die ein Peptid, bestehend aus einer Aminosäuresequenz mit mindestens 70 % Identität zu SEQ ID NO: 1 oder bestehend aus SEQ ID NO: 1 oder SEQ ID NO: 2, zur Verwendung bei der Vorbeugung oder Behandlung von posttraumatischer Belastungsstörung, akutem Belastungssyndrom und/oder allgemeinem Anpassungssyndrom umfasst.

2. Zusammensetzung, welche die Nukleotidsequenz, welche für die Aminosäuresequenz nach Anspruch 1 kodiert, zur Verwendung bei der Vorbeugung oder Behandlung von posttraumatischer Belastungsstörung, akutem Belastungssyndrom und/oder allgemeinem Anpassungssyndrom umfasst.

3. Zusammensetzung zur Verwendung nach einem der Ansprüche 1 oder 2, die zusätzlich einen Hilfsstoff und/oder einen verträglichen pharmazeutischen Träger und/oder ein Adjuvans oder beliebige Kombinationen davon umfasst.

4. Zusammensetzung zur Verwendung nach einem der Ansprüche 1 bis 3, die zusätzlich ein Internalisierungmittel, ausgewählt aus der Liste, bestehend aus: Cyclodextrin, Poloxameren, Rezeptorbindungspeptiden von Acetylcholin, Viren, VLP (virusähnlichen Partikeln), Cereport® (RMP-7), Mizellen, Liposomen, Polyethylen-Glykolaten, PEG-PLA, PEG-PCL, PEG-PHDCA, TAT 47-57 (SEQ ID NO: 3), TAT 48-60 (SEQ ID NO: 4), Antp (Penetratin, SEQ ID NO: 5) oder Pep-1 (SEQ ID NO: 6), umfasst.

5. Zusammensetzung zur Verwendung nach einem von Anspruch 1 bis 4, wobei sie in einer an die orale, parenterale, intramuskuläre, intraarterielle, intravenöse, subkutane, epidurale, intraperitoneale, intradermale oder intravesikuläre angepassten Verabreichungsform angeboten wird.

6. Zusammensetzung zur Verwendung nach einem der Ansprüche 1 bis 5, wobei die Zusammensetzung während 1 bis 7 Tagen verabreicht wird, vorzugsweise zwischen 1 bis 2 Tagen, vorzugsweise nach dem Trauma.

7. Zusammensetzung zur Verwendung nach einem der Ansprüche 1 bis 6, wobei die Vorbeugung oder die Behandlung bei einem Menschen durchgeführt wird.

8. Zusammensetzung zur Verwendung nach einem von Anspruch 1 bis 7, wobei die Vorbeugung vor der Gedächtniskonsolidierung durchgeführt wird.

9. Zusammensetzung zur Verwendung nach einem der Ansprüche 1 bis 8, wobei die Behandlung nach einer Gedächtniskonsolidierung unter Verwendung eines Gedächtnisrekonsolidierungs-Protokolls durchgeführt wird.

## Revendications

1. Composition qui comprend un peptide constitué d'une séquence d'acides aminés avec au moins 70 % d'identité avec SEQ ID NO: 1, ou constitué de SEQ ID NO: 1 ou SEQ ID NO: 2, pour son utilisation dans la prévention ou le traitement du trouble de stress post-traumatique, du syndrome de stress aigu et/ou du syndrome d'adaptation générale.

2. Composition qui comprend la séquence nucléotidique qui codifie la séquence d'acides aminés décrite dans la revendication 1 pour son utilisation dans la prévention ou le traitement du trouble de stress post-traumatique, du syndrome de stress aigu et/ou du syndrome d'adaptation générale.

3. Composition pour son utilisation selon l'une quelconque des revendications 1 ou 2 qui comprend en outre un excipient et/ou un vecteur pharmaceutiquement acceptable et/ou un adjuvant, ou toute combinaison de ceux-ci.

4. Composition pour son utilisation selon l'une quelconque des revendications 1 à 3 qui comprend en outre un agent d'internalisation choisi dans la liste constituée de : cyclodextrine, poloxamères, peptides de liaison aux récepteurs d'acétylcholine, virus, VLPs (pseudo-particules virales), Cereport® (RMP-7), micelles, liposomes, polyéthylène glycols, PEG-PLA, PEG-PCL, PEG-PHDCA, TAT 47-57 (SEQ ID NO: 3), TAT 48-60 (SEQ ID NO: 4), Antp (pénétratine, SEQ ID NO: 5) ou Pep-1 (SEQ ID NO: 6).

5. Composition pour son utilisation selon l'une quelconque des revendications 1 à 4, dans laquelle elle se présente sous une forme adaptée pour l'administration par voie orale, parentérale, intramusculaire, intraartérielle, intraveineuse, sous-cutanée, péridurale, intradermique, intrapéritonéale ou intravésiculaire.

6. Composition pour son utilisation selon l'une quelconque des revendications 1 à 5, dans laquelle la composition est administrée pendant 1 à 7 jours, de préférence entre 1 à 2 jours, de préférence après le traumatisme.

7. Composition pour son utilisation selon l'une quelconque des revendications 1 à 6 dans laquelle la prévention ou le traitement est effectué chez un être humain.

8. Composition pour son utilisation selon l'une quelconque des revendications 1 à 7 dans laquelle la prévention est effectuée avant la consolidation de la mémoire.

9. Composition pour son utilisation selon l'une quelconque des revendications 1 à 8 dans laquelle le traitement est effectué après la consolidation de la mémoire en utilisant un protocole de reconsolidation de la mémoire.
